# EUROPEAN PATENT SPECIFICATION

(11) **EP 2 061 873 B1**
(45) Date of publication and mention of the grant of the patent: **23.11.2011**
(21) Application number: 07838279.3
(22) Date of filing: 13.09.2007
(51) Int. Cl.: C12N 5/00, A61L 27/38

(54) **CELL DELIVERY MATRICES**
ZELLFREISETZUNGSMATRIZEN
MATRICES D'ADMINISTRATION DE CELLULES

(30) Priority: 21.09.2006 US 846468 P
(43) Date of publication of application: 27.05.2009
(73) Proprietor: Tissue Genesis, Honolulu, HI 96813 (US)
(72) Inventor: BOLAND, Eugene, D., Honolulu, HI 96825 (US); WILLIAMS, Stuart, K., Harrods Creek, KY 40027 (US); KOSNIK, Paul, E., Honolulu, HI 96813 (US)
(74) Representative: Ilgart, Jean-Christophe
(86) International application number: PCT/US2007/020048
(87) International publication number: WO 2008/036209

(56) References cited:
- WO-A-00/62833
- WO-A-94/00560
- WO-A-2004/067704
- WO-A-2006/084040
- US-A- 5 246 451
- US-A- 5 372 945
- US-A- 5 478 739
- US-A1- 2003 216 812
- US-A1- 2004 044 403
- US-A1- 2004 078 073
- STRAUER B.E.: 'Repair of Infarcted Myocardium by Autologous Intracoronary Monocuclear Bone Marrow Cell Transplantation in Humans' CIRCULATION vol. 106, 2002, pages 1913 - 1918, XP002364839

## Description

### FIELD OF THE INVENTION

The present disclosure relates generally to compositions and methods for improving the efficacy of cell based therapies through use of a composition that significantly mitigates migration of the cells from the site of delivery. More specifically, the present disclosure relates to cell delivery matrices that localize adipose derived endothelial cells and improve adherence of the endothelial cells to the target tissue, body cavity, or joint.

### BACKGROUND OF THE INVENTION

In recent years, numerous therapies have been developed utilizing a variety of stem cells, presaging an emerging new specialty called regenerative medicine that promises to harness stem cells from embryonic and somatic sources to provide replacement cell therapies for genetic, malignant, and degenerative conditions. Adipose derived endothelial cells are pluripotent stem cells, having the ability to differentiate into smooth muscle or other types of cells, as described in Oliver Kocher and Joseph A. Madri, Modulation of Actin mRIVAs in Cultured Vascular Cells By Matrix Componenls and TGF-β, In Vitro Cellular & Developmental Biology, Vol. 25, No. 5. May 1989. As such, these cells are useful in retention or restoration of cardiac function in acute and chronic ischemia. Cells within adipose tissue can differentiate into cells expressing a cardiomyocytic or endothelial phenotype, as well as angiogenic and antiapoptotic growth factors.

Direct injection or transplantation of cells may effectively restore small areas of damage, but to reconstruct severe damage to injured tissue, resulting from major coronary artery blockage, for example, will require extensive therapy with numerous differentiated cells. Such therapy is enhanced by maintaining endothelial cells at a target site for a therapeutically effective period of time, which may be from hours to days. In some embodiments, a therapeutically effective period of time is weeks to months.

### SUMMARY OF THE INVENTION

Cell delivery matrices are described that maintain local delivery of adipose derived endothelial cells and other therapeutic agents to a target tissue, body cavity, or joint. The cell delivery matrix may be a three-dimensional matrix scaffold comprising fibrin derived from the patient's own body. The cell delivery matrix used in the methods of the invention may be degradable, bioabsorbable or non-degradable. In an embodiment, the cell delivery matrix is an artificial, FDA-approved synthetic polymer. In an embodiment, the cell delivery matrix comprises expanded polytetrafluoroethylene (ePTFE). In another embodiment, the cell delivery matrix comprises polyethyleneterephthalate (PET). The cell delivery matrix may be biocompatible and semi-permeable. The surface of the cell delivery matrix may comprise an immobilized adhesion molecule.

The present disclosure provides regenerative therapies comprising implanting in the subject cell delivery matrices localizing adipose derived endothelial cells. The cell delivery matrices maintain the adipose derived endothelial cells at the target for a therapeutically effective amount of time. The adipose derived endothelial cells can be allogenic or syngenic to the subject. The endothelial cells may be delivered alone or in combination with other therapeutic agents.

A skilled artisan will appreciate that the subject of the present invention may be any animal, including amphibians, birds, fish, mammals, and marsupials, but is preferably a mammal (e.g., a human; a domestic animal, such as a cat, dog, monkey, mouse, and rat; or a commercial animal, such as a cow, horse or pig). Additionally, the subject of the present invention may be of any age, including a fetus, an embryo, a child, and an adult.

### BRIEF DESCRIPTION OF THE FIGURES

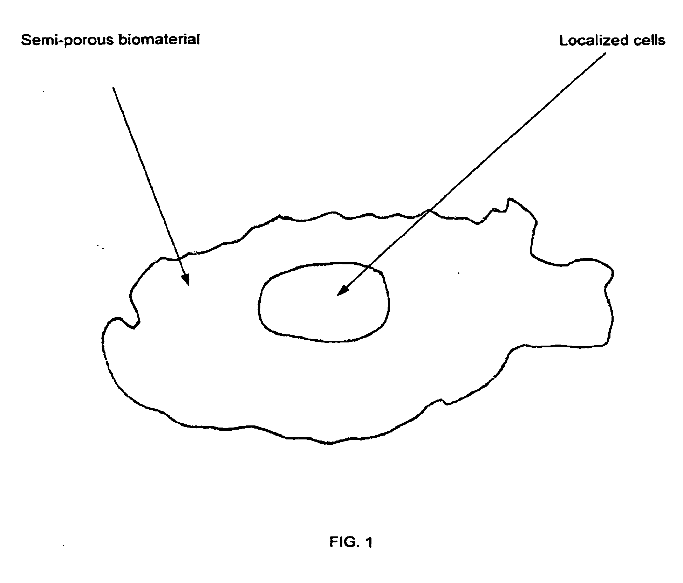

FIG. 1 depicts a cell delivery matrix. Arrows indicate localized endothelial cells and the semi-porous biomaterial.

### DETAILED DESCRIPTION

Those of ordinary skill in the art will realize that the following detailed description is illustrative only and is not intended to be in any way limiting. Other embodiments will readily suggest themselves to such skilled persons having the benefit of this disclosure.

As used herein and in the appended claims, the singular forms "a," "an," and "the" include plural references unless the content clearly dictates otherwise. All publication, patent applications, patents, and other references mentioned herein are incorporated by reference in their entirety. Additionally, the section headings used herein are for organizational purposes only and are not to be construed as limiting the subject matter descnbed.

U.S. Patent No. 5,372,945, discloses methods and devices that may be used for the ready isolation of large quantities of endothelial cells having the ability to differentiate into smooth muscle. According to an embodiment, subcutaneous fat is removed from a patient using modified liposuction techniques and transferred to a self-contained, closed device where the fat can be stored under sterile conditions until needed. The fat is sterilely transferred to a digestion device where it is initially washed to remove red blood cells and other debris, followed by a controlled collagenase digestion for about 20 minutes at about 37°C. The fat slurry is then transferred to an endothelial cell isolation device, again under sterile conditions, where endothelial cells sediment into an isolation device, allowing automatic retrieval of the isolated endothelial cells. The cell suspension is then sterilely transferred to a processing unit wherein the cells are rapidly filtered onto the graft surface under sterile conditions. The endothelial cell isolation and deposition process requires only about 40 minutes for completion. Following an incubation period, the graft is ready for implantation into the patient. The system yields endothelial cell product in numbers acceptable for subsequent high density seeding, e.g., in a range of about 5.14 X 10⁶ to 4.24 X 10⁷ cells from 50 cc of fat, and adherence to the graft surface. The apparatus deposits cells along the entire length and diameter of the graft consistently, with no significant difference in cell concentration as compared by analysis of variance.

As depicted in FIG. 1, after isolation these cells may then be localized by a cellular matrix. The cell delivery matrix that localizes the endothelial cells may be a three-dimensional culture, which is liquid, gel, semi-solid, or solid at 25°C. The three-dimensional culture may be biodegradable or non-biodegradable.

The cell delivery matrix used in the methods of the invention may be comprised of any degradable, bioabsorbable or non-degradable, biocompatible polymer. Exemplary three-dimensional culture materials include polymers and hydrogels comprising collagen, fibrin, chitosan, MATRIGEL, polyethylene glycol, dextrans including chemically crosslinkable or photocrosslinkable dextrans, and the like. In an embodiment, the three-dimensional culture comprises allogeneic components, autologous components, or both allogeneic components and autologous components. In an embodiment, the three-dimensional culture comprises synthetic or semi-synthetic materials. In an embodiment, the three-dimensional culture comprises a framework or support, such as a fibrin-derived scaffold. The term scaffold is used herein to include a wide variety of three-dimensional frameworks, for example, but not limited to a mesh, grid, sponge, foam, or the like.

The term "polymer" is also used herein in the broad sense and is intended to include a wide range of biocompatible polymers, for example, but not limited to, homopolymers, copolymers, block polymers, cross-linkable or crosslinked polymers, photoinitiated polymers, chemically initiated polymers, biodegradable polymers, nonbiodegradable polymers, and the like. In other embodiments, the prevascularized construct comprises a polymer matrix that is nonpolymerized, to allow it to be combined with a tissue, organ, or engineered tissue in a liquid or semi-liquid state, for example, by injection. In certain embodiments, the prevascularized construct comprising liquid matrix may polymerize or substantially polymerize "in situ." In certain embodiments, the prevascularized construct is polymerized or substantially polymerized prior to injection. Such injectable compositions are prepared using conventional materials and methods know in the art, including, but not limited to, Knapp et al., Plastic and Reconstr. Surg. 60:389 405, 1977; Fagien, Plastic and Reconstr. Surg. 105:362 73 and 2526 28, 2000; Klein et al., J. Dermatol. Surg. Oncol. 10:519 22, 1984; Klein, J. Amer. Acad. Dermatol. 9:224 28, 1983; Watson et al., Cutis 31:543 46, 1983; Klein, Dermatot. Clin. 19:491 508, 2001; Klein, Pedriat. Dent. 21:449 50, 1999; Skorman, J. Foot Surg. 26:511 5, 1987; Burgess, Facial Plast. Surg. 8:176 82, 1992; Laude et al., J. Biomech. Eng. 122:231 35,2000; Frey et al., J. Urol. 154:812 15, 1995; Rosenblatt et al., Biomaterials 15:985 95, 1994; Griffey et al., J. Biomed. Mater. Res. 58:10 15, 2001; Stenburg et al., Scfand. J. Urol. Nephrol. 33:355 61,1999; Sclafani et al., Facial Plast. Surg. 16:29 34, 2000; Spira et al., Clin. Plast. Surg. 20:181 88, 1993; Ellis et al., Facila Plast. Surg. Clin. North Amer. 9:405 11, 2001; Alster et al., Plastic Reconstr. Surg. 105:2515 28, 2000; and U.S. Pat. Nos. 3,949,073 and 5,709,854.

A cell delivery matrix may comprise collagen, including contracted and non-contracted collagen gels, hydrogels comprising, for example, but not limited to, fibrin, alginate, agarose, gelatin, hyaluronate, polyethylene glycol (PEG), dextrans, including dextrans that are suitable for chemical crosslinking, photocrosslinking, or both, albumin, polyacrylamide, polyglycolyic acid, polyvinyl chloride, polyvinyl alcohol, poly(n-vinyl-2-pyrollidone), poly(2-hydroxy ethyl methacrylate), hydrophilic polyurethanes, acrylic derivatives, pluronics, such as polypropylene oxide and polyethylene oxide copolymer, or the like. The fibrin or collagen may be autologous or allogeneic with respect to the patient. The matrix may comprise non-degradable materials, for example, but not limited to, expanded polytetrafluoroethylene (ePTFE), polytetrafluoroethylene (PTFE), polyethyleneterephthalate (PET), poly(butylenes terephthalate (PBT), polyurethane, polyethylene, polycabonate, polystyrene, silicone, and the like, or selectively degradable materials, such as poly (lactic-co-glycolic acid; PLGA), PLA, or PGA. (See also, Middleton et al., Biomaterials 21:2335 2346, 2000; Middleton et al., Medical Plastics and Biomaterials, March/April 1998, at pages 30 37; Handbook of Biodegradable Polymers, Domb, Kost, and Domb, eds., 1997, Harwood Academic Publishers, Australia; Rogaila, Minim. Invasive Surg. Nurs. 11:67 69, 1997; Klein, Facial Plast. Surg. Clin. North Amer. 9:205 18, 2001; Klein et al., J. Dermatol. Surg. Oncol. 11:337 39, 1985; Frey et al., J. Urol. 154:812 15, 1995; Peters et al., J. Biomed. Mater. Res. 43:422 27, 1998; and Kuijpers et al., J. Biomed. Mater. Res. 51:136 45, 2000).

The surface of the cell delivery matrix may comprise an immobilized adhesion molecule, as described in US Patent No. 5,744,515. In certain embodiments the immobilized adhesion molecule is selected from the group consisting of fibronectin, laminin, and collagen. The adhesion molecules may be immobilized to the surface, including the pores of the surface, of the matrix by means of photochemistry.

The cell delivery matrix, in addition to localizing endothelial cells, may localize at least one cytokine, at least one chemokine, at least one antibiotic, such as an antimicrobial agent, at least one drug, at least one analgesic agent, at least one anti-inflammatory agent, at least one immunosuppressive agent, or various combinations thereof. The at least one cytokine, at least one antibiotic, at least one drug, at least one analgesic agent, at least one anti-inflammatory agent, at least one immunosuppressive agent, or various combinations thereof may comprise a controlled-release format, such as those generally known in the art, for example, but not limited to, Richardson et al., Nat. Biotechnol. 19:1029 34, 2001.

Exemplary cytokines include angiogenin, vascular endothelial growth factor (VEGF, including, but not limited to VEGF-165), interleukins, fibroblast growth factors, for example, but not limited to, FGF-1 and FGF-2, hepatocyte growth factor, (HGF), transforming growth factor beta (TGF-.beta.), endothelins (such as ET-1, ET-2, and ET-3), insulin-like growth factor (IGF-1), angiopoietins (such as Ang-1, Ang-2, Ang-3/4), angiopoietin-like proteins (such as ANGPTL1, ANGPTL-2, ANGPTL-3, and ANGPTL-4), platelet-derived growth factor (PDGF), including, but not limited to PDGF-AA, PDGF-BB and PDGF-AB, epidermal growth factor (EGF), endothelial cell growth factor (ECGF), including ECGS, platelet-derived endothelial cell growth factor (PD-ECGF), placenta growth factor (PLGF), and the like. Cytokines, including recombinant cytokines, and chemokines are typically commercially available from numerous sources, for example, R & D Systems (Minneapolis, Minn.); Endogen (Woburn, Wash.); and Sigma (St. Louis, Mo.). The skilled artisan will understand that the choice of chemokines and cytokines for incorporation into particular prevascularized constructs will depend, in part, on the target tissue or organ to be vascularized or revascularized.

In certain embodiments, the cell delivery matrix further localizes at least one genetically engineered cell. Descriptions of exemplary genetic engineering techniques can be found in, among other places, Ausubel et al., Current Protocols in Molecular Biology (including supplements through March 2002), John Wiley & Sons, New York, N.Y., 1989; Sambrook et al., Molecular Cloning: A Laboratory Manual, 2.sup.nd Ed., Cold Spring Harbor Laboratory Press, Cold Spring Harbor, N.Y., 1989; Sambrook and Russell, Molecular Cloning: A Laboratory Manual, 3.sup.rd Ed., Cold Spring Harbor Laboratory Press, Cold Spring Harbor, N.Y.,2001; Beaucage et al., Current Protocols in Nucleic Acid Chemistry, John Wiley & Sons, New York, N.Y., 2000 (including supplements through March 2002); Short Protocols in Molecular Biology, 4.sup.th Ed., Ausbel, Brent, and Moore, eds., John Wiley & Sons, New York, N.Y., 1999; Davis et al., Basic Methods in Molecular Biology, McGraw Hill Professional Publishing, 1995; Molecular Biology Protocols (see the highveld.com website), and Protocol Online (protocol-online.net). Exemplary gene products for genetically modifying the genetically engineered cells of the invention include, plasminogen activator, soluble CD4, Factor VIII, Factor IX, von Willebrand Factor, urokinase, hirudin, interferons, including alpha-, beta- and gamma-interferon, tumor necrosis factor, interleukins, hematopoietic growth factor, antibodies, glucocerebrosidase, adenosine deaminase, phenylalanine hydroxylase, human growth hormone, insulin, erythropoietin, VEGF, angiopoietin, hepatocyte growth factor, PLGF, and the like.

In certain embodiments, a cell delivery matrix further comprises appropriate stromal cells, stem cells, or combinations thereof. As used herein, the term "stem cells" includes traditional stem cells, progenitor cells, preprogenitor cells, reserve cells, and the like. Exemplary stem cells include embryonic stem cells, adult stem cells, pluripotent stem cells, neural stem cells, liver stem cells, muscle stem cells, muscle precursor stem cells, endothelial progenitor cells, bone marrow stem cells, chondrogenic stem cells, lymphoid stem cells, mesenchymal stem cells, hematopoietic stem cells, central nervous system stem cells, peripheral nervous system stem cells, and the like. Descriptions of stem cells, including method for isolating and culturing them, may be found in, among other places, Embryonic Stem Cells, Methods and Protocols, Turksen, ed., Humana Press, 2002; Weisman et al., Annu. Rev. Cell. Dev. Biol. 17:387 403; Pittinger et al., Science, 284:143 47, 1999; Animal Cell Culture, Masters, ed., Oxford University Press, 2000; Jackson et al., PNAS 96(25):14482 86, 1999; Zuk et al., Tissue Engineering, 7:211 228, 2001 ("Zuk et al."); Atala et al., particularly Chapters 33 41; and U.S. Pat. Nos. 5,559,022, 5,672,346 and 5,827,735. Descriptions of stromal cells, including methods for isolating them, may be found in, among other places, Prockop, Science, 276:71 74, 1997; Theise et al., Hepatology, 31:235 40, 2000; Current Protocols in Cell Biology, Bonifacino et al., eds., John Wiley & Sons, 2000 (including updates through March, 2002); and U.S. Pat. No. 4,963,489.

Therapeutic agents that can also be localized by the cell delivery matrix may include Transforming Growth Factor beta (TGFβ) and TGF-β-related proteins for regulating stem cell renewal and differentiation.

Further therapeutic agents that may be used include anti-thrombogenic agents or other agents for suppressing stenosis or late restenosis such as heparin, streptokinase, urokinase, tissue plasminogen activator, anti-thromboxane B² agents, anti-B-thromboglobulin, prostaglandin E, aspirin, dipyridimol, anti-thromboxane A₂ agents, murine monoclonal antibody 7E3, triazolopyrimidine, ciprostene, hirudin, ticlopidine, nicorandil, and the like. Anti-platelet derived growth factor may be used as a therapeutic agent to suppress subintimal fibromuscular hyperplasia at an arterial stenosis site, or any other inhibitor of cell growth at the stenosis site may be used.

Other therapeutic agents that may be used in conjunction with endothelial cells may comprise a vasodilator to counteract vasospasm, for example an antispasmodic agent such as papaverine. The therapeutic agents may be vasoactive agents generally such as calcium antagonists, or alpha and beta adrenergic agonists or antagonists. Additionally, the therapeutic agent may be an anti-neoplastic agent such as 5-fluorouracil or any known anti-neoplastic agent, preferably mixed with a controlled release carrier for the agent, for the application of a persistent, controlled release anti-neoplastic agent to a tumor site.

The therapeutic agent may be an antibiotic, which may be applied to an infected stent or any other source of localized infection within the body. Similarly, the therapeutic agent may comprise steroids for the purpose of suppressing inflammation or for other reasons in a localized tissue site.

Additionally, glucocorticosteroids or omega-3 fatty acids may be localized by the cell delivery matrix, particularly for stenosis applications. Any of the therapeutic agents may include controlled release agents to prolong the persistence.

The therapeutic agent may constitute any desired mixture of individual pharmaceuticals of the like, for the application of combinations of active agents. The pharmaceutical agent may support the survival of the cell (e.g., a carbohydrate, a cytokine, a vitamin, etc.). The cell delivery matrix can be delivered to the target tissue, body cavity, or joint by any local delivery means known in the art. Applicant's provisional application 60/841,009, entitled "Catheter for Cell Delivery," incorporated herein by reference in its entirety, discloses methods and apparatuses suitable for local delivery of the cell delivery matrices of the present disclosure. In an embodiment, the cell delivery system used to deliver the cells locally comprises a catheter. The catheter may comprise an inner bladder and an outer perforated bladder that permits localized delivery of stem cells. The inner bladder may be expanded through the use of a pressure conduit in order to deploy a stent. Cell matrices comprising endothelial cells may be introduced between the inner and outer bladder. The inner bladder may be further expanded in order to exert pressure on the outer perforated bladder to advance the cells though the apertures of the outer bladder. The inner bladder may remain pressurized to hold the outer bladder against the vessel wall, thereby directing the cells to specific target sites. In an embodiment, a three-dimensional matrix scaffold comprising fibrin is delivered locally without cells, in accordance with the methods disclosed in Application Number 60/841,009.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as the presently preferred embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

## Claims

1. A composition comprising a three-dimensional, semi-permeable, bio-compatible cell delivery matrix 5 enveloping a pellet of microvessel endothelial cells obtainable by the method according to claim 2, the matrix configured to localize the endothelial cells and to maintain the endothelial cells at a target of a subject for a therapeutically effective amount of time. 10

2. A method for preparing a cell delivery matrix comprising:
providing a collecting vessel for processing tissue to produce an endothelial cell product, the 15 vessel having a rinsing and digesting chamber in fluid communication with a separate waste chamber and an isolation chamber connected to the rinsing and digesting chamber, wherein a screen separates at least the rinsing and digesting chamber and the waste 20 chamber;
introducing adipose tissue into the rinsing and digesting chamber;
introducing rinsing solution into the rinsing and digesting chamber;
orienting the vessel to screen the tissue to be processed of rinsing solution passed into the waste chamber;
introducing collagenase into the rinsing and digesting chamber;
heating the tissue and the enzyme for a sufficient time and temperature while agitating the rinsing and digesting chamber to digest the tissue with the enzyme;
centrifuging the vessel to transfer cells from the digested tissue from the rinsing and digesting chamber into the isolation chamber;
isolating the cells as a pellet of microvessel endothelial cells; and
enveloping the pellet of microvessel endothelial cells with a semi-permeable matrix.

3. The composition of claim 1 or the method of claim 2, wherein the matrix comprises fibrin.

4. The composition of claim 1 or the method of claim 2, wherein the matrix comprises collagen.

5. The composition of claim 1 or the method of claim 2, wherein the matrix comprises a polymer.

6. The composition or method of claim 5, wherein the polymer is expanded polytetrafluoroethylene.

7. The composition or method of claim 5, wherein the polymer is polyethyleneterephthalate.

8. Matrix as defined in claim 1 or prepared according to the method of claim 2, for use in implantation in a subject, wherein the endothelial cells are allogenic to the subject.

9. Matrix as defined in claim 1 or prepared according to the method of claim 2, for use in implantation in a subject, wherein the endothelial cells are syngenic to the subject.

10. Matrix as defined in claim 1 or prepared according to the method of claim 2, for use in implantation in a subject, wherein said matrix comprises fibrin derived from said subject.

## Patentansprüche

1. Zusammensetzung, umfassend eine dreidimensionale, semipermeable, biokompatible Zellabgabematrix, die ein Pellet von Mikrogefäß-Endothelzellen umhüllt, erhältlich durch das Verfahren gemäß Anspruch 2, wobei die Matrix konfiguriert ist, um die Endothelzellen zu lokalisieren und um die Endothelzellen an einem Ziel eines Patienten über einen therapeutisch wirksamen Zeitraum hinweg zu erhalten.

2. Verfahren zum Herstellen einer Zellabgabematrix, umfassend
Bereitstellen eines Gewinnungsgefäßes zum Verarbeiten von Gewebe, um ein Endothelzellprodukt herzustellen, wobei das Gefäß eine Spül- und Verdaukammer in Fluidverbindung mit einer getrennten Abfallkammer und einer Isolierungskammer, verbunden mit der Spül-und Verdaukammer, aufweist, wobei eine Scheibe mindestens die Spül-und Verdaukammer und die Abfallkammer trennt;
Einbringen von Fettgewebe in die Spül- und Verdaukammer;
Einbringen von Spüllösung in die Spül- und Verdaukammer;
Orientieren des Gefäßes, um das Gewebe, das verarbeitet werden soll, von Spüllösung, die in die Abfallkammer geleitet wird, abzuschirmen;
Einbringen von Collagenase in die Spül- und Verdaukammer;
Erwärmen des Gewebes und des Enzyms über einen ausreichend langen Zeitraum hinweg und bei einer ausreichenden Temperatur unter Schütteln der Spül- und Verdaukammer, um das Gewebe mit dem Enzym zu verdauen;
Zentrifugieren des Gefäßes, um Zellen von dem verdauten Gewebe aus der Spül- und Verdaukammer in die Isolierungskammer zu übertragen;
Isolieren der Zellen als Pellet von Mikrogefäß-Endothelzellen und
Umhüllen des Pellets von Mikrogefäß-Endothelzellen mit einer semipermeablen Matrix.

3. Zusammensetzung nach Anspruch 1 oder Verfahren nach Anspruch 2, wobei die Matrix Fibrin umfasst.

4. Zusammensetzung nach Anspruch 1 oder Verfahren nach Anspruch 2, wobei die Matrix Collagen umfasst.

5. Zusammensetzung nach Anspruch 1 oder Verfahren nach Anspruch 2, wobei die Matrix ein Polymer umfasst.

6. Zusammensetzung oder Verfahren nach Anspruch 5, wobei es sich bei dem Polymer um expandiertes Polytetrafluorethylen handelt.

7. Zusammensetzung oder Verfahren nach Anspruch 5, wobei es sich bei dem Polymer um Polyethylenterephthalat handelt.

8. Matrix, definiert wie in Anspruch 1 oder hergestellt gemäß dem Verfahren nach Anspruch 2, zur Verwendung bei der Implantation in einen Patienten, wobei die Endothelzellen zu dem Patienten allogen sind.

9. Matrix, definiert wie in Anspruch 1 oder hergestellt gemäß dem Verfahren nach Anspruch 2, zur Verwendung bei der Implantation in einen Patienten, wobei die Endothelzellen zu dem Patienten syngen sind.

10. Matrix, definiert wie in Anspruch 1 oder hergestellt gemäß dem Verfahren nach Anspruch 2, zur Verwendung bei der Implantation in einen Patienten, wobei die Matrix von dem Patienten abgeleitetes Fibrin umfasst.

## Revendications

1. Une composition comprenant une matrice d'administration de cellules, tridimensionnelle, semi-perméable, biocompatible, enveloppant un culot de cellules endothéliales de microvaisseaux susceptible d'être obtenu par un procédé selon la revendication 2, la matrice configurée pour localiser les cellules endothéliales et maintenir les cellules endothéliales au niveau d'une cible d'un sujet pendant un laps de temps thérapeutiquement efficace.

2. Un procédé pour préparer une matrice d'administration de cellules comprenant les étapes consistant à :
fournir un récipient de collecte pour traiter un tissue afin de produire un produit à base de cellules endothéliales, le recipient ayant une chambre de rinçage et de digestion en communication fluidique avec une chambre d'élimination séparée et une chambre d'isolement connectée à la chambre de rincage et de digestion, dans lequel un tamis sépare au moins la chambre de rinçage et de digestion et la chambre d'isolement ;
introduire un tissue adipeux dans la chambre de rinçage et de digestion ;
introduire une solution de rinçage dans la chambre de rinçage et de digestion ;
orienter le récipient pour isoler le tissue à traiter de la solution de rinçage envoyée dans la chamber d'élimination ;
introduire de la collagénase dans la chambre de rinçage et de digestion ;
chauffer le tissue et l'enzyme pendant un temps et à une température suffisant(e), pendant que la chambre de rinçage et de digestion est agitée, pour digérer le tissu avec l'enzyme ;
centrifuger le récipient pour transférer les cellules du tissu digéré de la chambre de rinçage et de digestion dans la chambre d'isolement ;
isoler les cellules sous forme d'un culot de de cellules endothéliales de microvaisseaux ; et
envelopper le culot de cellules endothéliales de microvaisseaux avec une matrice semi-perméable.

3. La composition de la revendication 1 ou le procédé de la revendication 2, dans lequel/laquelle la matrice comprend de la fibrine.

4. La composition de la revendication 1 ou le procédé de la revendication 2, dans lequel/laquelle la matrice comprend du collagène.

5. La composition de la revendication 1 ou le procédé de la revendication 2, dans lequel/laquelle la matrice comprend un polymère.

6. La composition ou le procédé de la revendication 5, dans lequel/laquelle le polymère est du polytétrafluoroéthylène expansé.

7. La composition ou le procédé de la revendication 5, dans lequel/laquelle le polymère est du polyéthylènetéréphthalate.

8. Matrice telle que définie dans la revendication 1 ou préparée selon le procédé de la revendication 2, pour utilisation dans l'implantation dans un sujet, dans laquelle les cellules endothéliales sont allogéniques au sujet.

9. Matrice telle que définie dans la revendication 1 ou préparée selon le procédé de la revendication 2, pour utilisation dans l'implantation dans un sujet, dans laquelle les cellules endothéliales sont syngéniques au sujet.

10. Matrice telle que définie dans la revendication 1 ou préparée selon le procédé de la revendication 2, pour utilisation dans l'implantation dans un sujet, dans laquelle ladite matrice comprend de la fibrine dérivée dudit sujet.
